# EUROPÄISCHE PATENTSCHRIFT

(11) **EP 0 751 163 B1**
(45) Veröffentlichungstag und Bekanntmachung des Hinweises auf die Patenterteilung: **29.05.2002**
(21) Anmeldenummer: 96109686.4
(22) Anmeldetag: 17.06.1996
(51) Int. Cl.: C08G 18/79, C08G 18/80, C07D 251/34, C09D 175/04

(54) **Verfahren zur Herstellung von Lösungen von Isocyanuratgruppen aufweisenden Polyisocyanaten mit verringertem Restmonomergehalt und ihre Verwendung**
Process for the preparation of solutions of polyisocyanates containing isocyanurate groups having reduced content of residual monomer and their use
Procédé de préparation de solutions de polyisocyanates contenant des groupes isocyanurate à teneur réduite en monomère résiduel et leur utilisation

(30) Priorität: 29.06.1995 DE 19523657
(43) Veröffentlichungstag der Anmeldung: 02.01.1997
(73) Patentinhaber: BAYER AG, 51368 Leverkusen (DE)
(72) Erfinder: Schmalstieg, Lutz, Dr., 50676 Köln (DE); Dieris, Carl-Gerd, Dr., 41539 Dormagen (DE); Kremer, Wolfgang, 47647 Kerken (DE); Riberi, Bernd, Dr., 51519 Odenthal (DE)

(56) Entgegenhaltungen:
- EP-A- 0 416 338
- EP-A- 0 585 835

## Beschreibung

Die vorliegende Erfindung betrifft ein verbessertes Verfahren zur Herstellung von Lösungen von Isocyanuratgruppen aufweisenden Polyisocyanaten auf Basis von Diisocyanatotoluolen mit einem Gehalt an freiem Diisocyanatotoluol von weniger als 0,1 %. Die Erfindung betrifft auch die Verwendung derartiger Lösungen zur Herstellung von Zweikomponenten Polyurethanlacken.

Isocyanuratgruppen aufweisende Polyisocyanate auf Basis von 2,4- und gegebenenfalls 2,6-Diisocyanatotoluol stellen wertvolle Bestandteile für Zweikomponenten Polyurethanlacke für die Holz- und Möbellackierung dar. Die Herstellung der modifizierten Polyisocyanate geschieht im allgemeinen durch Teiltrimerisierung der Isocyanatgruppen von 2,4- und gegebenenfalls 2,6-Diisocyanatotoluol in 30 bis 70 gew.-%iger Lösung in geeigneten Lacklösemitteln. Derartige Polyisocyanate werden beispielhaft beschrieben in DE-OS 2 414 413, DE-OS 2 452 532 und DE-OS 3 928 503.

In den genannten Veröffentlichungen werden auch Lösungen von Isocyanuratgruppen aufweisenden Polyisocyanaten mit einem Restgehalt an freiem Diisocyanatotoluol von 0,1 % beschrieben. Derart niedrige Restmonomerengehalte können durch einen hohen Trimerisationsgrad erzielt werden, müssen jedoch durch eine drastisch erhöhte Viskosität erkauft werden, was unter ökologischen Gesichtspunkten von Nachteil ist.

Eine weitere Möglichkeit besonders niedrige Restmonomergehalte zu erzielen ist die Nachurethanisierung gemäß den Ausführungen der DE-OS 2 414 413. Doch auch die Nachurethanisierung ist mit einer deutlichen Viskositätserhöhung verbunden.

Die Aufgabe der vorliegenden Erfindung bestand folglich darin, Lösungen von Isocyanuratgruppen aufweisenden Polyisocyanaten auf Basis von Diisocyanatotoluol herzustellen, die sich durch einen Restgehalt an freiem Diisocyanatotoluol von weniger als 0,1 Gew.-% einerseits und eine niedrige Viskosität andererseits auszeichnen.

Diese Aufgabe konnte dadurch gelöst werden, daß die bei der Herstellung der Trimerisate als Ausgangsdiisocyanate eingesetzten Diisocyanatotoluole mit sehr geringen Mengen längerkettiger Monoalkohole urethanisiert werden und die urethanisierten Ausgangsdiisocyanate anschließend in Form von 40 bis 70 gew.-%iger Lösung in geeigneten Lacklösemitteln einer Teiltrimerisierung unterzogen wurden, bis der Gehalt an freiem Ausgangsdiisocyanat unter 0,1 Gew.-% abgesunken ist.

Gegenstand der Erfindung ist ein verbessertes Verfahren zur Herstellung von Isocyanuratgruppen aufweisenden Polyisocyanaten in gegenüber Isocyanatgruppen inerten Lacklösungsmitteln, durch Umsetzung von 2,4-Diisocyanatotoluol, gegebenenfalls im Gemisch mit bis zu 20 Gew.-%, bezogen auf Gemisch, 2,6-Diisocyanatotoluol mit einem einwertigen Alkohol und anschließende Trimerisierung eines Teils der nach der Urethanisierung vorliegenden Isocyanatgruppen in Gegenwart eines die Trimerisierung von Isocyanatgruppen beschleunigendes Katalysators und Abbruch der Trimerisierungsreaktion durch Zugabe eines Katalysatorgiftes, dadurch gekennzeichnet, daß man
(i) 0,2 bis 1,5 % der NCO-Gruppen mit einem einwertigen Alkohol R-OH umsetzt, wobei R für einen linearen oder verzweigten, gesättigten oder ungesättigten aliphatischen Kohlenwasserstoffrest mit 6 bis 22 Kohlenstoffatomen steht,
(ii) die urethanisierten Diisocyanate der Trimerisierungsreaktion in Form von 40 bis 70 gew.-%igen Lösungen in gegenüber Isocyanatgruppen inerten Lacklösungsmittel zuführt und
(iii) die Trimerisierungsreaktion durch Zugabe eines Katalysatorengifts abbricht, nachdem der Gehalt des Reaktionsgemisches an freiem Diisocyanatotoluol unter 0,1 Gew.-% liegt.

Gegenstand der Erfindung ist auch die Verwendung der nach diesem Verfahren erhaltenen Lösungen von Isocyanuratgruppen aufweisenden Polyisocyanaten zur Herstellung von Zweikomponenten-Polyurethanlacken.

Es ist aus der DE-OS 3 928 503 bereits bekannt, 2,4- und gegebenenfalls 2,6-Diisocyanatotoluol durch Urethanisierung eines Teils der Isocyanatgruppen und anschließende Trimerisierung eines weiteren Teils der Isocyanatgruppen zu modifizieren um hierdurch eine verbesserte Aromatenverdünnbarkeit zu erzielen. Gemäß der Lehre dieser Veröffentlichung werden zur Urethanisierung vergleichsweise hohe Anteile an Diisocyanat der Urethanmodifizierung unterworfen (2,5 % bis 7 % der NCO-Gruppen). Durch diese Maßnahme verschlechtert sich aber das Trocknungsverhalten der Polyisocyanate dramatisch, so daß die Produkte der DE-OS 3 928 503 den diesbezüglichen Anforderungen der Praxis nicht genügten.

Im Hinblick auf die Lehre der DE-OS 3 928 503 muß es als ausgesprochen überraschend angesehen werden, daß die erfindungsgemäße Modifizierung mit sehr geringen Mengen an Monoalkohol einen so deutlichen Effekt bezüglich Monomerengehalt und Viskosität der Verfahrensprodukte bewirkt, da die Autoren der zitieren Veröffentlichung diesen Effekt trotz der Verwendung von vergleichsweise hohen Mengen an Alkoholen nicht erwähnen.

Das nachstehend näher beschriebene erfindungsgemäße Verfahren unterscheidet sich somit vom Verfahren der DE-OS 3 928 503 sowohl bezüglich der Aufgabenstellung als auch bezüglich der zur Lösung der unterschiedlichen Aufgabe ergriffenen Maßnahmen.

Ausgangsmaterial für das erfindungsgemäße Verfahren ist 2,4-Diisocyanatotoluol, gegebenenfalls im Gemisch mit bis zu 20 Gew.-%, bezogen auf Gemisch, 2,6-Diisocyanatotoluol. Die Verwendung von reinem 2,4-Diisocyanatotoluol ist bevorzugt.

Zumindest die zweite Stufe des erfindungsgemäßen Verfahrens erfolgt in Gegenwart von gegenüber Isocyanatgruppen inerten Lacklösungsmitteln. Derartige Lacklösungsmittel sind beispielsweise Ethylacetat, n-Butylacetat, Methylethylketon, Methylisobutylketon, Methoxypropylacetat oder Gemische derartiger Lösungsmittel.

Die erste Stufe des erfindungsgemäßen Verfahrens besteht in der Umsetzung von 0,2 bis 1,5 %, vorzugsweise 0,2 bis 1 % der Isocyanatgruppen der gelöst vorliegenden Ausgangsdiisocyanate mit einem einwertigen Alkohol oder einem Gemisch einwertiger Alkohole der nachstehend genannten Art.

Geeignete einwertige Alkohole sind solche der allgemeinen Formel

R-OH

worin R für einen linearen oder verzweigten, gesättigten oder ungesättigten aliphatischen Kohlenwasserstoffrest mit 6 bis 22, vorzugsweise 8 bis 18 Kohlenstoffatomen steht.

Beispiele für geeignete einwertige Alkohole sind 1-Hexanol, 1-Octanol, 2-Ethylhexanol, 1-Decanol, 1-Dodecanol, 1-Tetradecanol, Stearylalkohol, Linoleylalkohol, Oleylalkohol, Behenylalkohol. Besonders bevorzugt wird 1-Dodecanol eingesetzt.

Die erste Stufe des erfindungsgemäßen Verfahrens besteht in der Urethanisierung eines Teils der Isocyanatgruppen des Ausgangsdiisocyanats mit der genannten Alkoholkomponente, wobei 0,2 bis 1,5, vorzugsweise 0,2 bis 1 % der Isocyanatgruppen der Ausgangsdiisocyanate durch Umsetzung mit dem einwertigen Alkohol oder einem Gemisch aus verschiedenen einwertigen Alkoholen der beispielhaft genannten Art urethanisiert werden. Diese Modifizierung des Diisocyanats mit den Monoalkoholen erfolgt bei etwa 0 bis 120°C, insbesondere bei 20 bis 80°C in Substanz oder in Lösung, wobei als Lösungsmittel solche der bereits oben beispielshaft genannten Art verwendet werden, die im Anschluß an die Urethanisierung als Reaktionsmedium für die Teiltrimerisierung dienen. Im Falle der Urethanisierung in Abwesenheit von Lösungsmitteln werden die teilurethanisierten Ausgangsdiisocyanate vor der Trimerisierung in einem Lösungsmittel oder Lösungsmittelgemisch der beispielhaft genannten Art gelöst.

Die anschließende Teiltrimerisierung der Isocyanatgruppen erfolgt auf katalytischem Wege bei 20 bis 80°C, wobei 40- bis 70 gew.-%ige Lösungen der anurethanisierten Ausgangsdiisocyanate zum Einsatz gelangen.

Als Trimerisierungskatalysatoren kommen grundsätzlich alle bekannten Trimerisierungskatalysatoren des Standes der Technik wie beispielsweise Phophine, Alkalisalze, Alkalialkoholate, tert.-Amine u. dgl. in Betracht. Vorzugsweise werden jedoch Mannich-Basen der bereits in DE-OS 24 52 532 genannten Art als Trimerisierungskatalysatoren verwendet. Die Trimerisierung wird so lange fortgesetzt, bis der Gehalt des Reaktionsgemisches an Urethangruppen- und Isocyanuratgruppenfreiem Ausgangsdiisocyanat unter 0,1 Gew.-%, bezogen auf Lösung, abgesunken ist. Dies entspricht etwa der Trimerisierung von 50 bis 60 % der nach der Urethanisierung noch vorliegenden Isocyanatgruppen.

Der Abbruch der Trimerisierungsreaktion erfolgt durch Zugabe eines Katalysatorengifts, wobei als Katalysatorengifte beispielsweise Schwefel (im Falle der Verwendung von Phosphinen als Katalysatoren) oder Alkylierungsmittel wie beispielsweise Toluolsulfonsäuremethylester (bei der bevorzugten Verwendung von Mannich-Basen als Katalysatoren) oder auch Acylierungsmittel wie beispielsweise Benzoylchlorid Verwendung finden können.

Die beim erfindungsgemäßen Verfahren als Verfahrensprodukte anfallenden Lösungen von Isocyanuratgruppen aufweisenden Polyisocyanaten weisen einen NCO-Gehalt von 12 bis 17 Gew.-% bezogen auf Feststoff, auf.

Die nach dem erfindungsgemäßen Verfahren hergestellten Produkte zeigen bei vergleichbarem Festkörpergehalt und vergleichbarem Restmonomergehalt wesentlich niedrigere Viskositäten als die Produkte des Standes der Technik. Dies wird durch einen nachstehend angeführten Vergleichsversuch belegt.

Die nach dem erfindungsgemäßen Verfahren hergestellten Polyisocyanate eignen sich hervorragend als Härter für 2-Komponenten-Polyurethanlacke. Sie zeigen eine gute Verträglichkeit mit anderen handelsüblichen Lackbindemitteln und eine gute Verdünnbarkeit mit aromatischen Solventien.

In 2-Komponenten-Polyurethanlacken vermitteln die nach dem erfindungsgemäßen Verfahren hergestellten Produkte hervorragende Trocknungseigenschaften, vergleichbar mit denen entsprechender nicht-urethanmodifizierter Polyisocyanate.

In den nachfolgenden Beispielen beziehen sich alle Prozentangaben soweit nicht anders vermerkt auf Gewichtsprozente. Die Viskositäten wurden rotationsviskosimetrisch als Einpunktmessung gemäß DIN 53 019 bestimmt (HAAKE-Viskosimeter. VT02, Drehkörper Nr. 1).

In den nachfolgenden Beispielen wird als Katalysatorlösung eine 40 Gew.-%ige Lösung in Xylol einer Mannich-Base der Formel 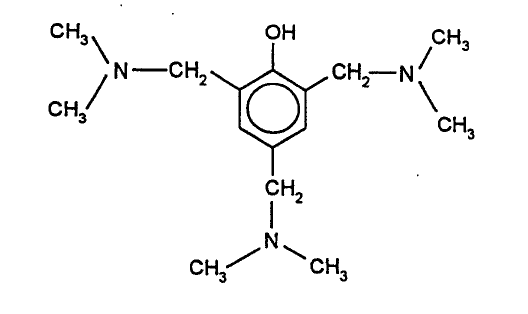 verwendet.

### Beispiel 1

477 g 2,4-Diisocyanatotoluol werden vorgelegt und auf 60°C erwärmt. Bei dieser Temperatur wird 3,7 g geschmolzenes n-Dodecanol zugegeben. Es wird bei 60°C urethanisiert bis ein NCO-Gehalt von 47,7 % erreicht ist. Danach wird mit 480 g Butylacetat verdünnt, wodurch sich die Mischung auf 40°C abkühlt. Bei 40°C wird die Trimerisation durch Zugabe von 1 g Katalysatorlösung gestartet Es wird bei einer Temperatur von 40°C trimerisiert. Man katalysiert im 12 Stunden Rhythmus mit jeweils 0,4 g Katalysatorlösung nach und trimerisiert bis nach ca. 48 h ein NCO-Gehalt von 7,5 % erreicht ist.

Zur Vernichtung des Katalysators fügt man 2 g Toluolsulfonsäuremethylester zu und erhitzt 1 Stunde auf 80°C. Man erhält eine klare, blaßgelb gefärbte Lösung mit folgenden Kenndaten:

| | |
|---|---|
| Konzentration | ca. 50 % |
| Viskosität | 700 mPas (23°C) (D = 80,7 s⁻¹) |
| NCO-Gehalt | 7,5 % |
| freies Diisocyanat | 0,03 % |
| Urethangruppengehalt | 0,36 Mol-% der NCO-Gruppen urethanisiert. |

### Beispiel 2

Man verfährt wie in Beispiel 1, setzt aber als Monoalkohol 4,4 g n-Decanol ein. Man erhält eine Lösung eines Isocyanurat-Polyisocyanats mit folgenden Kenndaten:

| | |
|---|---|
| Konzentration | ca. 50 % |
| Viskosität | 800 mPas (23°C) (D= 80,7 s⁻¹) |
| NCO-Gehalt | 7,4 % |
| freies Diisocyanat | < 0,03 % (Nachweisgrenze) |
| Urethangruppengehalt | 0,5 Mol- % der NCO-Gruppen urethanisiert. |

### Beispiel 3

### Nicht erfindungsgemäßes Vergleichsbeispiel

Man verfährt wie in Beispiel 1, verzichtet aber auf eine Modifizierung mit Alkohol.

| | |
|---|---|
| Konzentration | ca. 50 % |
| Viskosität | 1700 mPas (23°C) (D = 80,7 s⁻¹) |
| NCO-Gehalt | 8,0 % |
| freies Diisocyanat | 0,15 % |

### Beispiel 4

### Nicht erfindungsgemäßes Vergleichsbeispiel

Man verfährt wie in Beispiel 1, verzichtet wiederrum auf eine Modifizierung mit Alkohol.

| | |
|---|---|
| Konzentration | ca. 50 % |
| Viskosität | 6800 mPas (23°C) (D = 80,7 s⁻¹) |
| NCO-Gehalt | 7,6 % |
| freies Diisocyanat | 0,04 % |

### Beispiel 5

Eigenschaftsvergleich eines erfindungsgemäßen 2-Komponenten-Polyurethanlacks mit einem nicht erfindungsgemäßen.

Die Polyisocyanatlösungen aus Beispiel 1 und Beispiel 3 werden jeweils im NCO/OH-Verhältnis 0,7:1 mit einem handelsüblichen Polyol mit einem OH-Gehalt von 4,0 % (®Desmophen 1300, Handelsprodukt der Bayer AG) kombiniert, wobei durch Verdünnen mit Butylacetat ein Festkörpergehalt von 45 % eingestellt wird.

Es werden Filme auf Glasplatten gerakelt mit einer Naßfilmdicke von 180 µm.

| | Polyisocyanatlösung aus Beispiel 1 | | | | Polyisocyanatlösung aus Beispiel 3 | | | |
|---|---|---|---|---|---|---|---|---|
| Trocknung (min) (handtrocken, griffest) | 33 | | | | 33 | | | |
| Auslaufzeit (s) im DIN-4-Becher bei 23°C | | | | | | | | |
| sofort | 17 | | | | 18 | | | |
| 2 h | 23 | | | | 24 | | | |
| 4 h | 36 | | | | 39 | | | |
| 6 h | 72 | | | | 77 | | | |
| Pendelhärte nach | | | | | | | | |
| 4 h | 119 | | | | | | | |
| 6 h | 150 | | | | 118 | | | |
| 1 d | 168 | | | | 148 | | | |
| 4 d | 182 | | | | 167 | | | |
| 7 d | 183 | | | | 171 | | | |
| Anlösbarkeit nach | *a | b | b | d | a | b | c | d |
| 8 h | **3 | 3 | 4 | 5 | 3 | 3 | 4 | 5 |
| 1d | 1 | 1 | 1 | 5 | 1 | 1 | 2 | 5 |
| 4d | 0 | 0 | 0 | 5 | 0 | 0 | 1 | 5 |
| 7d | 0 | 0 | 0 | 5 | 0 | 0 | 0 | 5 |

| | | | | | | | | |
|---|---|---|---|---|---|---|---|---|
| *a) Toluol, b) Butylacetat, c) MPA, d) Aceton | | | | | | | | |
| ** 0) unverändert, bester Wert, 5) angelöst, schlechtester Wert | | | | | | | | |

## Patentansprüche

1. Verfahren zur Herstellung von Isocyanuratgruppen aufweisenden Polyisocyanaten in gegenüber Isocyanatgruppen inerten Lacklösungsmitteln durch Umsetzung von 2,4-Diisocyanatotoluol, gegebenenfalls im Gemisch mit bis zu 20 Gew.-%, bezogen auf Gemisch, 2,6-Diisocyanatotoluol mit einem einwertigen Alkohol und anschließende Trimerisierung eines Teils der nach der Urethanisierung vorliegenden Isocyanatgruppen in Gegenwart eines die Trimerisierung von Isocyanatgruppen beschleunigenden Katalysators und Abbruch der Trimerisierungsreaktion durch Zugabe eines Katalysatorgiftes, **dadurch gekennzeichnet, daß** man
i) 0,2 % bis 1,5 % der NCO-Gruppen mit einem einwertigen Alkohol R-OH umsetzt, wobei R für einen linearen oder verzweigten, gesättigten oder ungesättigten aliphatischen Kohlenwasserstoffrest mit 6 bis 22 Kohlenstoffatomen steht,
ii) die urethanisierten Diisocyanate der Trimerisierungsreaktion in Form von 40 bis 70 gew.-%igen Lösungen in gegenüber Isocyanatgruppen inerten Lacklösungsmitteln zuführt und
iii) die Trimerisierungsreaktion durch Zugabe eines Katalysatorgiftes abbricht, nachdem der Gehalt des Reaktionsgemisches an freiem Diisocyanatotoluol unter 0,1 Gew.-% liegt.

2. Verfahren zur Herstellung von Isocyanuratgruppen aufweisenden Polyisocyanaten gemäß Anspruch 1, **dadurch gekennzeichnet, daß** 0,2 % bis 1 % der NCO-Gruppen mit einem einwertigen Alkohol R-OH umgesetzt werden.

3. Verfahren zur Herstellung von Isocyanuratgruppen aufweisenden Polyisocyanaten gemäß Anspruch 1 und 2, **dadurch gekennzeichnet, daß** ausschließlich 2,4-Diisocyanatotoluol verwendet wird.

4. Verfahren zur Herstellung von Isocyanuratgruppen aufweisenden Polyisocyanaten gemäß Anspruch 1 bis 3, **dadurch gekennzeichnet, daß** man als einwertige Alkohole ROH lineare Alkanole mit 8 bis 18 Kohlenstoffatomen einsetzt.

5. Verwendung der gemäß Anspruch 1 bis 4 erhaltenen Lösungen von Isocyanuratgruppen aufweisenden Polyisocyanaten zur Herstellung von Zweikomponenten Polyurethanlacken.

## Claims

1. A process for preparing polyisocyanates with isocyanurate groups in lacquer solvents which are inert towards isocyanate groups by reacting 2,4-diisocyanatotoluene, optionally mixed with up to 20 wt.%, with respect to the mixture, of 2,6-diisocyanatotoluene, with a monohydric alcohol and then trimerising some of the isocyanate groups present after urethanisation in the presence of a catalyst for accelerating the trimerisation of isocyanate groups and terminating the trimerisation reaction by adding a catalyst poison, **characterised in that**
(i) 0.2 to 1.5 % of the NCO groups are reacted with a monohydric alcohol R-OH, wherein R represents a linear or branched, saturated or unsaturated, aliphatic hydrocarbon group with 6 to 22 carbon atoms,
(ii) the urethanised diisocyanates are taken to the trimerisation reaction in the form of 40 to 70 wt.% -strength solutions in lacquer solvents which are inert towards isocyanate groups and
(iii)the trimerisation reaction is terminated by adding a catalyst poison, after which the concentration of free diisocyanatotoluene in the reaction mixture is less than 0.1 wt.%.

2. A process for preparing polyisocyanates with isocyanurate groups according to Claim 1, **characterised in that** 0.2 % to 1 % of the NCO groups are reacted with monohydric alcohol R-OH.

3. A process for preparing polyisocyanates with isocyanurate groups according to Claims 1 and 2, **characterised in that** exclusively 2,4-diisocyanatotoluene is used.

4. A process for preparing polyisocyanates with isocyanurate groups according to Claims 1 to 3, **characterised in that** linear alkanols with 8 to 18 carbon atoms are used as monohydric alcohols ROH.

5. Use of the solutions of polyisocyanates with isocyanurate groups obtained according to Claims 1 to 4 to produce two-component polyurethane lacquers.

## Revendications

1. Procédé de préparation de polyisocyanates présentant des groupes isocyanurates dans des solvants pour laque inertes vis-à-vis des groupes isocyanates, en faisant réagir du 2,4-diisocyanatotoluène, éventuellement en mélange avec jusqu'à 20% en poids, rapporté au mélange, de 2,6-diisocyanatotoluène avec un alcool monovalent et ensuite en faisant la trimérisation d'une partie des groupes isocyanates existant après l'uréthanisation en présence d'un catalyseur accélérant la trimérisation des groupes isocyanates et en terminant la réaction de trimérisation par addition d'un poison pour catalyseur, **caractérisé en ce que**
(i) on fait réagir 0,2% à 1,5% des groupes NCO avec un alcool monovalent R-OH, où R est un radical hydrocarboné aliphatique saturé ou insaturé, linéaire ou ramifié avec 6 à 22 atomes de carbone,
(ii) on amène des diisocyanates uréthanisés de la réaction de trimérisation sous forme de solution de 40 à 70% en poids dans des solvants pour laque inertes par rapport aux groupes isocyanates et
(iii) on termine la réaction de trimérisation par addition d'un poison de catalyseur, après quoi le mélange réactionnel présente une quantité en diisocyanatotoluène libre inférieure à 0,1% en poids.

2. Procédé de préparation de polyisocyanates présentant des groupes isocyanurates selon la revendication 1, **caractérisé en ce qu'**on fait réagir 0,2% à 1% des groupes NCO avec un alcool monovalent R-OH.

3. Procédé de préparation de polyisocyanates présentant des groupes isocyanurates selon la revendication 1 et 2, **caractérisé en ce qu'**on utilise exclusivement du 2,4-diisocyanatotoluène.

4. Procédé de préparation de polyisocyanates présentant des groupes isocyanurates selon la revendication 1 à 3, **caractérisé en ce qu'**on utilise comme alcools monovalents des alcools linéaires ROH avec 8 à 18 atomes de carbone.

5. Utilisation de polyisocyanates présentant des groupes isocyanurates de solutions obtenues selon la revendication 1 à 4 pour la préparation de laques de polyuréthanes à deux composants.
